# EUROPEAN PATENT APPLICATION

(11) **EP 1 586 332 A1**
(43) Date of publication of application: **19.10.2005**
(21) Application number: 04702856.8
(22) Date of filing: 16.01.2004
(51) Int. Cl.: A61K 45/00, A61K 38/05

(54) **COMBINED USE OF CARNOSINASE INHIBITOR WITH L-CARNOSINES AND COMPOSITION**

(30) Priority: 20.01.2003 JP 2003010639
(71) Applicant: Innovative Vision Products, Inc., New Castle, DE 19810 (US); Hamari Chemicals, Ltd., Osaka-shi, Osaka 533-0024 (JP)
(72) Inventor: BABIZHAYEV, M.A., Innovative Vision Products, Inc., 127434 Moscow (RU); MEGURO, Kanji, Nishinomiya-shi, Hyogo 662-0825 (JP)
(74) Representative: Teipel, Stephan
(86) International application number: PCT/JP2004/000351
(87) International publication number: WO 2004/064866

(57) **Abstract**

The present invention provides combined use of a carnosinase inhibitor with L-carnosine and its related substance, and a composition containing the same, which are useful for treatment or prevention of various diseases, improvement of health conditions, improvement of exercise ability, improvement of skin health, prevention of the side effects of alcohol drinking and the like in mammals including human.

## Description

### Technical Field

This invention relates to combined use of a carnosinase inhibitor with L-carnosine and its related substance, and a composition containing the same. The combined use of a carnosinase inhibitor with L-carnosine and its related substance, and a composition containing the same are useful in the filed of pharmaceutical compositions, cosmetics, foods and beverages.

### Background Art

Free radicals and the peroxidative processes caused by them are believed to be one of the causes of the structural and functional degradations of human and other mammalian tissues during aging. It is important for prevention or minimization of the aging processes and related pathologies, namely for longevity, to maintain the high concentration of natural antioxidant molecules (free radical scavengers) inside and around the body's various tissues and cells, and in the body's liquids including the blood plasma and blood stream in order to supply organs, tissues and cells with the antioxidant molecules.

L-Carnosine (β-alanyl-L-histidine) (I) has been found to be one of the most abundant (1-20 mM) nitrogenous compounds present in the non-protein fraction of vertebrate skeletal muscles (e.g. non-patent literature 1,2) and certain other tissues, including olfactory epithelium, and bulbs (e.g. non-patent literature 3) and also the crystalline lens (e.g. non-patent literature 4).

Some L-carnosine-related natural substances such as N-acetyl-L-carnosine, L-anserine (β-alanyl-L-1-methylhistidine) and its acyl derivatives, L-balenine (β-alanyl-L-3-methylhistidine) and its acetyl derivatives, L-homocarnosine (γ-aminobutyryl-L-histidine) and its acetyl derivative, and carcinine (β-alanylhistamine) and its acetyl derivatives have been also reported (e.g. non-patent literature 5,6,7) to be present at millimolar concentrations in several mammalian tissues, including skeletal muscles, cardiac tissue and brain, although there are interesting differences in their distribution, activities and metabolic transformation (e.g. non-patent literature 8).

L-Carnosine and its related natural substances mentioned above have been known to be among the most important natural antioxidant agents acting both in the lipid phase of the cellular or biological membranes and in the aqueous environment, to protect lipids and water-soluble molecules like proteins (including enzymes), DNA and other essential macromolecules from oxidative damage mediated by reactive oxygen species and lipid peroxides (e.g. non-patent literature 9, 10, 11, 12).

Various antioxidant enzymes such as SOD (superoxide dismutase) or catalase can only react with their substrates in the aqueous environment. On the other hand, previously published data suggest that L-carnosine and its related natural substances have excellent potential to act as natural antioxidants which scavenge hydroxyl radical, superoxide and singlet oxygen, resulting in protection of lipids from peroxidation (e.g. non-patent literature 13, 14).

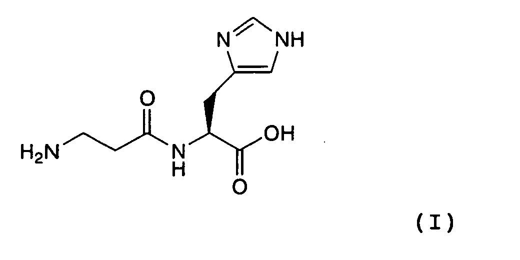

Further detailed explanation on the physiological properties of L-carnosine and related compounds is as follows:
1) L-Carnosine and its naturally occurring related substances protect the proprietary cellular and tissue antioxidant enzymes (like SOD) and systems (like glutathione and ceruloplasmin) from inactivation. L-Carnosine, though water soluble, potentiates the antioxidant affect of lipid soluble alpha tocopherol during lipid peroxidation in the liver microsomes. It is thus a major protector of the liver cytochrome P-450 system. L-Carnosine is also the only known antioxidant to significantly protect cellular chromosomes from oxidative damage. These antioxidant effects are thought to be beneficial to prevent atherosclerosis, ischemic diseases (e.g., brain infarction, transient ischemic arrest, stroke, angina pectoris, cardiac infarction, etc.), formation and progression of cataracts, and aging of the muscles and the skin. L-Carnosine is also known to ameliorate the neuro-degenerative diseases of the brain (e.g., Alzheimer's disease, dementia, epilepsy, etc.). Recently it is postulated that a dysfunctioning of the anti-oxidant system such as L-carnosine, glutathione and superoxide dismutase (SOD), causes overstimulation of the post-synaptic glutamate receptors (NMDA and AMPA) leading to the expression and progression of schizophrenia, Parkinson's disease, Huntington's chorea, and amyotrophic lateral sclerosis (e.g. non-patent literature 15). It is thought that supplement of L-carnosine is a useful way to prevent and to treat these diseases.
2) L-Carnosine is the most effective anti-glycating agent ever found and is known to prevent the so-called Maillard reaction in both the direct glycation of proteins and the advanced glycation such as cross-linking of the proteins. It is expected, therefore, that L-carnosine prevents not only diabetic complications such as diabetic retinopathy, diabetic neuropathy, diabetic nephropathy, etc., but also aging of various tissues such as vascular tissues (atherosclerosis, retinopathy, etc.), skin (wrinkles), lenses (cataracts), and so on. Very recently, it has also been reported that a carcinogenic and toxic substance acrylamide was formed in a variety of foods during cooking through the Maillard reaction (e.g. non-patent literature 16,17). Addition of L-carnosine to pre-cooked foods or foodstuffs is therefore expected as a useful mean to prevent the formation of acrylamide.
3) L-Carnosine has a membrane-stabilizing and tissue-repairing effects and is effective in protecting and repairing the ulcerative diseases of the gastrointestinal tract(e.g. gastric ulcer and duodenal ulcer) and the skin. It is also effective to heal a wound and a burn.
4) L-Carnosine and its related natural substances have anti-ischemic effects not only in the brain, but also in the heart. L-Carnosine has been shown to increase the strength of heart contractility by enhancing calcium response in the heart cells. L-Carnosine injections have been shown to be effective emergency treatment for ischemic crises.
5) L-Carnosine has been shown to play an important role to control the acid-base balance of the muscles when a large quantity of H⁺ is produced in association with lactic acid accumulation during high-intensity exercise. Thus L-carnosine plays a role to prevent the fatigue of the muscles leading to the improvement of the exercise-performance ability. It protects muscle cell membranes from oxidation under the acidic conditions of muscular exercise. According to a recent report, the L-carnosine concentration in the muscle was significantly correlated with the mean power body mass (e.g. non-patent literature 18). L-Carnosine has been shown to dramatically improve exercise recovery (but does not increase performance, which means that it is not an "ergogenic aid," but rather facilitates the anabolic response to exercise). Carnosine has been shown to quickly restore muscle contraction capability after fatigue.
6) Recently, it is said that L-carnosine is likely to be the true substrate of nitric oxide synthase. The supplement of L-carnosine stimulates the synthesis of nitric oxide (NO) which is an internal blood vessel relaxing material, and is expected to reduce blood pressure, prevent ischemic diseases like angina pectoris and improve penile erectile dysfunction.
7) L-Carnosine can act as a scavenger of acetaldehyde (non-patent literature 19) which is the main product of ethanol metabolism upon the consumption of alcohol-containing drinks (whisky, cognac, vodka, beer, sake, wine, etc.). Thus L-carnosine protects the liver, the brain and muscles from the toxic effect of the acetaldehyde. L-Carnosine due to its wound healing property is also effective to cure and prevent the ulcers in the gastro-intestinal tract which can be exacerbated during the alcohol consumption.
8) The chronic form of alcoholic skeletal myopathy is characterized by selective atrophy of Type II fibers and affects up to two thirds of all alcohol misusers. L-Carnosine prevents the atrophy of the fibers. Carnosine protects the brain from both lipid peroxidation and damage caused by excessive alcohol intake.
9) L-Carnosine has immunostimulating property. It protects the immune system from immunosuppression by hydrocortisone, anti-tumor drugs, and many other immunosuppressive drugs.
10) L-Carnosine has the following benefits for keeping health and youth: L-Carnosine and related compounds increase the number of the cell divisions (Hayflick limit) and may be useful for elongation of the life span and for longevity (e,g, non-patent literature 20). L-Carnosine has a rejuvenating effect on connective tissue cells and a wound healing effect. L-Carnosine has been shown to rejuvenate cells approaching senescence by extending the life of those cells which will continue to divide with the frequency typical of youth. In tissue cultures supplemented with L-carnosine, cells retain a youthful appearance and have an extended cellular life span. This ability for L-carnosine to increase cellular life span holds true even for old cells. One study showed a 67% increase in cellular life span with L-carnosine supplementation. This property has been proved in vivo by using mice. The mice supplemented with L-carnosine lived an average of 20% longer than non-treated mice, and kept a healthy state twice more than non-treated mice when they reached an old age. In humans, L-carnosine levels decline with age. Muscle L-carnosine concentration decreases 63% during age 10 to age 70.

On the other hand, however, exogenous carnosine, even when topically administered to the eye, does not accumulate in the tissues, because it is easily excreted in urine or destroyed by the enzyme called carnosinase, which is present in blood plasma, aqueous humor of the anterior eye chamber, liver, kidney and other tissues, but not in the muscles (e.g. non-patent literature 21,22)) and probably in the lens (e.g. non-patent literature 23,24. L-Carnosine-related natural substances such as N-acetyl-L-carnosine, L-anserine, N-acetyl-L-anserine, L-balenine, L-homocarnosine, N-acetyl-L-homocarnosine and carcinine can be more or less the substrates of carnosinase to be hydrolyzed and deactivated, although the rates of the hydrolysis are slower than that of L-carnosine (e.g. non-patent literature 25).

As mentioned above, it is predicted that a shortage of L-carnosine and/or its related natural substances in the body as a result of aging or malnutrition, for instance, causes a variety of dysfunction of the body of humans and other mammals. The supplement of L-carnosine or its natural related substances is therefore thought to be useful to treat or prevent these dysfunction or the resulting diseases. It is also expected that an excessive supplement of L-carnosine and/or its related natural substances becomes meaningful to cure these diseases, to improve exercise performance, to prevent aging, to care the skin, to improve the quality of life (QOL), and so on. Although L-carnosine is already used as a dietary supplement or a cosmetic in some countries, these are not thought to be very effective, because, as mentioned above, L-carnosine is easily hydrolyzed to β-alanine and L-histidine by an enzyme called carnosinase (in its serum and tissue forms), resulting in the complete loss of the physiological effects of L-carnosine. Since carnosinase is widely distributed in the lumen of the small intestine, in the plasma, and in the various tissues of humans and mammals, the administration of a large excess of L-carnosine regardless of the route of the treatment, i.e., oral, parenteral, or topical, has been recommended in order to attain the above-mentioned purposes, namely treatment of diseases, improvement of exercise performance, prevention of aging, skin care, improvement of QOL, and so on, by saturating the enzyme to prevent the complete hydrolysis of the L-carnosine administered. However, such a large excess treatment should be avoided not only from the practical and economical point of view, but also from the viewpoint of safety, in particular due to the risk of histamine release in tissues and blood plasma. When a large excess amount of L-carnosine is applied, excessive histamine may be formed in the body by the action of another enzyme histidine decarboxylase(s) on the excessive L-histidine released by carnosinase. Therefore, development of an alternative and safe way that makes the efficient delivery of L-carnosine or its related natural substance (s) to the target tissues possible without its degradation has long been waited.

On the other hand, there has been no literature concerning the use of carnosinase inhibitors to increase the delivery of L-carnosine or its related natural substances to the plasma or the target tissues or organs by preventing it from hydrolysis and deactivation. It is known that bestatin is a potent tissue carnosinase inhibitor (e.g. non-patent literature 26), and that accumulation of internal tissue L-carnosine is increased upon treatment with bestatin. However, it has not been known if bestatin can effectively help deliver the L-carnosine to the target tissues when L-carnosine and bestatin are administered simultaneously in vivo.
[Non-patent literature 1] K.G.Crush, Comp. Biochem. Physiol., Vol. 34, 3, 1970;
[Non-patent literature 2] A.A. Boldyrev, S.E. Severin, Adv. Enzyme Regul., Vol. 30, 175, 1990
[Non-patent literature 3] F. Margolis, Science, Vol. 184, 909, 1974
[Non-patent literature 4] Jay et al., Meeting Abstr. J. Physiol. London, Vol. 420, 155, 1990
[Non-patent literature 5] P.R. Carnegie et al., J. Chromatogr., Vol. 261, 153, 1983
[Non-patent literature 6] L. Flancbaum et al., Life Sci., Vol. 47, 1587, 1990
[Non-patent literature 7] M.A. Babizhayev et al., Biochemistry (Moscow), Vol.63, 620, 1998
[Non-patent literature 8] M. A. Babizhayev et al. , Biochem. J. , Vol.304, 509, 1994
[Non-patent literature 9] M.A. Babizhayev et al. , Biochem. J., Vol. 304, 509, 1994 and references thereof.
[Non-patent literature 10] M.A. Babizhayev et al., Biochemistry (Moscow), Vol. 63, 523, 1998
[Non-patent literature 11] M.A. Babizhayev et al., Clinica Chimica Acta, Vol. 254, 1, 1996
[Non-patent literature 12] J.H. Kang et al. , Molecules and Cells, Vol. 13, 107, 2002 and references thereof.
[Non-patent literature 13] T.A. Dahl, W.R. Midden, P.E. Hartman, Photochem. Photobiol., Vol. 47, 357, 1988
[Non-patent literature 14] M.A. Babizhayev et al. , Biochem. J., Vol. 304, 509, 1994 and references thereof
[Non-patent literature 15] Encephale, Vol. 28(2), 147, 2002
[Non-patent literature 16] D.S. Mottram, et al, Nature, Vol. 419, 448, 2002
[Non-patent literature 17] R.H. Stadler, et al., Nature, Vol. 419, 449, 2002
[Non-patent literature 18] Y. Suzuki et al., Japan. J of Physiol., Vol. 64, 199, 2002
[Non-patent literature 19] Ann. NY Acad. Sci., Vol. 854, 37, 1998
[Non-patent literature 20] R. Holliday, G.A. McFarland., Biochemistry (Moscow), Vol. 65, 843, 2000
[Non-patent literature 21] Jackson et al., Clin. Chim. Acta, Vol.196, 193, 1991 [Non-patent literature 22] Lenney et al., Biochem. J., Vol. 228, 653, 1985
[Non-patent literature 23] A.A. Boldyrev, A.Y. Dupin, M.A. Babizhayev, S.E. Severin, Biochem. Int., Vol. 15, 1105, 1987
[Non-patent literature 24] J.L. Jay et al., Meeting Abstr. J. Physiol. Lond., Vol. 420, 155, 1990
[Non-patent literature 25] A. Pegova et al., Comp. Biochem. Physiol. B. Biochem. Mol. Biol., Vol.127(4), 443, 2000
[Non-patent literature 26] S.C. Peppers, J.F. Lenney, Biol. Chem. Hoppe Seyler, Vol. 369, 1281, 1988)

### Disclosure of the Invention

This invention relates to the combined use of a carnosinase inhibitor and L-carnosine or its related substances and to the formulation thereof. This invention is useful for treatment and prevention of L-carnosine-related diseases, improvement of health conditions, improvement of exercise ability, prevention of aging, improvement of skin health, and prevention of the side effects of alcohol drinking.

The present inventors completed this invention by finding that it is useful to prevent or treat carnosine-related diseases of mammals when L-carnosine and a carnosinase inhibitor are used in combination. The inventors also found that such combined use and formulation thereof are useful for improvement of health conditions, improvement of exercise performance, prevention of aging, improvement skin health, and prevention of the side effects of alcohol drinking.

According to this invention, L-carnosine or its related substances administered orally, parenterally or topically (to the skin, eyes, mucous membrane of mouth or nose, respiratory tract, etc.) is delivered effectively to the plasma or the target tissues or organs with high bioavailability, when a carnosinase inhibitor is administered simultaneously through a route similar to the one used for L-carnosine or its related substances. Any combination of the compounds and the administration route for each compound may be possible. As the result, the dosage of L-carnosine or its natural derivative to be applied can be reduced or the physiological effects of L-carnosine can be potentiated markedly. It was also found that in order to increase the absorption of L-carnosine or its related substances and/or a carnosinase inhibitor, addition of at least one of the additives selected from cellulose derivatives, gelatin and polyvinylpyrrolidone is useful. This invention relates to these unique and effective methods.

Furthermore, this invention relates to a novel method for providing a new chemotherapy of cancer characterized by administering bestatin, a tissue carnosinase inhibitor, together with L-carnosine. Although bestatin itself has been known to have anti-cancer effect, the present inventors found that the anti-cancer effect was improved by the simultaneous administration of L-carnosine. This is based on the hitherto unknown, unexpected finding that the increase of L-carnosine level in the cancer cells results in the prevention of the division of the malignant cancer cells without affecting normal cells. The precise molecular basis of this anti-cancer activity of L-carnosine according to the data of the present inventors is related to the ferroxidase activity of L-carnosine in the malignant cancer cells, by which the deprivation of ferrous ion concentration in the malignant cancer cells occurs inducing their apoptosis, a prompt death of the malignant cancer cells.

The present inventors have made extensive studies on carnosinase inhibitors and discovered β-alanine (II), N-alkanoyl-β-alanine derivatives (III) and N-alkanoyl-L-carnosine derivatives (IV) to be safe and cheap carnosinase inhibitors for practical use.

Betastatin (V) or its ester derivative was also found to be a useful carnosinase inhibitor for the purpose of this invention as well as for cancer chemotherapy. 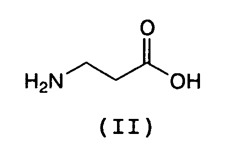 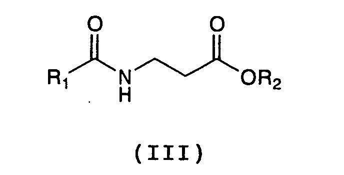 (R₁ and R₂ independently represent hydrogen atom or a lower alkyl group) 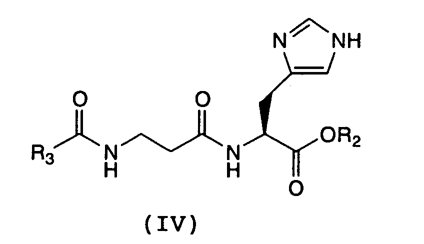 (R₂ represents hydrogen atom or a lower alkyl group, and R₃ represents an alkyl group of more than two carbon atoms) 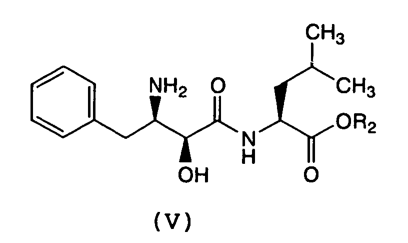 (R₂ represents hydrogen atom or a lower alkyl group, and R₃ represents an alkyl group of more than two carbon atoms)

Namely, this invention relates to:
(1) combined use of a carnosinase inhibitor with L-carnosine and its related substance to treat or prevent carnosine-related disorders and diseases of humans and other mammals,
(2) combined use of a carnosinase inhibitor with L-carnosine and its related substance to improve health conditions of humans and other mammals,
(3) combination of a carnosinase inhibitor with L-carnosine or its related substance to improve health conditions of the skin of humans and other mammals,
(4) Combined use of a carnosinase inhibitor with L-carnosine or its related substance to prevent adverse effects caused by drinking alcohol,
(5) the combined use described in (1) to (4), wherein at least one of the carnosinase inhibitors is selected from the group comprising of β-alanine (II), an N-alkanoyl-β-alanine derivative(III), an N-alkanoyl-L-carnosine derivative (IV) and bestatin (V), 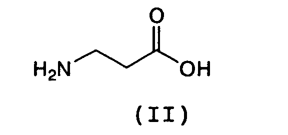 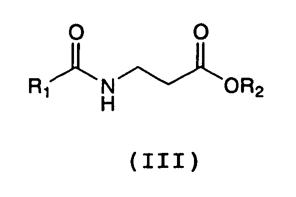 (R₁ and R₂ independently represent hydrogen atom or a lower alkyl group) 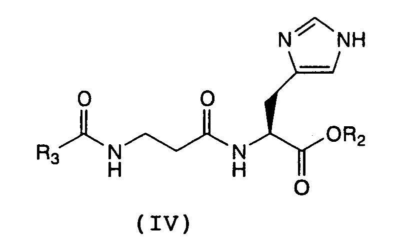 (R₂ represents hydrogen atom or a lower alkyl group, and R₃ represents an alkyl group of more than two carbon atoms) 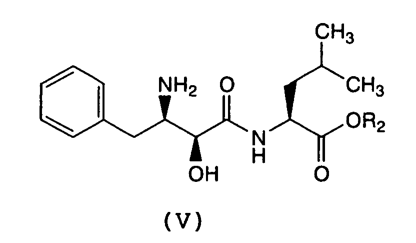 (R₂ represents hydrogen atom or a lower alkyl group)
(6) the combined use described in (1) to (4) to increase the concentration of L-carnosine or its related substance in the plasma and the cells of humans and other mammals to the physiologically effective levels,
(7) the combined use described in (1) to (4), wherein the L-carnosine-related substance is N-acetyl-L-carnosine, L-anserine, N-acetyl-L-anserine, L-balenine, L-homocarnosine, N-acetyl-L-homocarnosine or carcinine,
(8) the combined use described in (5), wherein the carnosinase inhibitor is β-alanine,
(9) the combined use described in (5), wherein N-alkanoyl-β-alanine (III) is N-acetyl-β-alanine which may be esterified by a lower alkyl group,
(10) the combined use described in (1) to (4), wherein at least one of the carnosinase inhibitors is selected from the group comprising of an α-amino acid derivative, γ-amino acid derivative, a thiol type reducing agent, a metal chelating agent, a hydrophobic vitamin with a branched hydrocarbon residue, manganese ion or zinc ion,
(11) the method of increasing the concentration of L-carnosine or its related substance in the plasma and the cells to the physiologically effective level, wherein at least one of the carnosinase inhibitors is selected from the group comprising of β-alanine (II), an N-alkanoyl-β-alanine derivative (III), an N-alkanoyl-L-carnosine derivative (IV), bestatin (V), an α-amino acid derivative, a γ-amino acid derivative, a thiol type reducing agent, a metal chelating agent, a hydrophobic vitamin with a branched hydrocarbon residue, manganese ion and zinc ion,
(12) the method described in (11), wherein N-acetyl-L-carnosine, L-anserine, N-acetyl-L-anserine, L-balenine, L-homocarnosine, N-acetyl-L-homocarnosine, or carcinine is used as an L-carnosine-related substance,
(13) the method described in (11), wherein the carnosinase inhibitor is β-alanine,
(14) a pharmaceutical composition containing L-carnosine or its related natural substance and at least one of the carnosinase inhibitors is selected from the group comprising of β-alanine (II), an N-alkanoyl-β-alanine derivative (III), N-alkanoyl-L-carnosine derivative (IV), bestatin (V), an α-amino acid derivative, a γ-amino acid derivative, a thiol type reducing agent, a metal chelating agent, a hydrophobic vitamin with a branched hydrocarbon residue, manganese ion and zinc ion,
(15) a health food or a dietary supplement composition containing L-carnosine or its related natural substance and at least one of the carnosinase inhibitors is selected from the group comprising of β-alanine (II), an N-alkanoyl-β-alanine derivative (III), an N-alkanoyl-L-carnosine derivative(IV), bestatin (V), an α-amino acid derivative, a γ-amino acid derivative, a thiol type reducing agent, a metal chelating agent, a hydrophobic vitamin with branched hydrocarbon structure, manganese ion and zinc ion,
(16) a health food or a dietary supplement composition described in (15), wherein the carnosinase inhibitor is β-alanine.
(17) a cosmetic or a skin-care composition containing L-carnosine or its related natural substance and at least one of the carnosinase inhibitors selected from the group comprising of β-alanine (II), an N-alkanoyl-β-alanine derivative (III), N-alkanoyl-L-carnosine derivative (IV), bestatin (V), an α-amino acid derivative, γ-amino acid derivative, a thiol type reducing agent, a metal chelating agent, a hydrophobic vitamin with branched hydrocarbon structure, manganese ion and zinc ion,
(18) alcoholic drinks containing L-carnosine or its related natural substance and a carnosinase inhibitor selected from the group comprising of β-alanine (II), an N-alkanoyl-β-alanine derivative (III), N-alkanoyl-L-carnosine derivative (IV), bestatin (V), an α-amino acid derivative, γ-amino acid derivative, a thiol type reducing agent, a metal chelating agent, a hydrophobic vitamin with branched hydrocarbon structure, manganese ion and zinc ion,
(19) the composition described in (14) to (17), wherein at least one of the additives selected from the group comprising of a cellulose derivative, gelatine and polyvinyl pyrrolidone is used to increase the absorption of L-carnosine or its related substance and/or the carnosinase inhibitor,
(20) the composition described in (14) to (18), wherein N-acetyl-L-carnosine, L-anserine, N-acetyl-L-anserine, L-balenine, L-homocarnosine, N-acetyl-L-homocarnosine, or carcinine is used as an L-carnosine-related substance,
(21) the composition described in (14) to (18), wherein the carnosinase inhibitor is β-alanine, and
(22) cancer chemotherapy characterized by treating cancer patients with L-carnosine or its related substance together with bestatin (V).

In the above formulae (III) and (IV), R₁ and R₂ may be the same or different and each represents hydrogen atom or a lower alkyl group with a straight chain or a branched chain, preferably having 1 to 6 carbon atoms such as methyl, ethyl, propyl, isopropyl, butyl, isobutyl, sec-butyl, tert-butyl, heptyl, hexyl, etc. R₃ in the above formula (IV) represents an alkyl group having more than two carbon atoms, which may be a straight or a branched one, such as ethyl, propyl, isopropyl, butyl, isobutyl, sec-butyl, tert-butyl, hexyl, heptyl, octyl, nonyl, decyl, undecyl, dodecyl, tridecyl, tetradecyl, pentadecyl, hexadecyl, heptadecyl, octadecyl, nonadecyl and eicosyl.

Such preferable N-alkanoyl-β-alanine derivatives (III) are exemplified by N-formyl-β-alanine, N-acetyl-β-alanine, N-propionyl-β-alanine, N-butyryl-β-alanine, N-(2-methylpropionyl)- β-alanine, N-pentanoyl-β-alanine, N-hexanoyl-β-alanine, N-heptanoyl-β-alanine, and their esters (e.g. lower alkyl esters with 1 to 6 carbon atoms such as methyl and ethyl esters). Above all, the most preferable and convenient one is N-acetyl-β-alanine. Above mentioned N-alkanoyl-β-alanine derivatives (III) may be known compounds or can be synthesized from β-alanine by per se known methods such as acylation or esterification.

Such preferable N-alkanoyl-L-carnosine derivatives (IV) are exemplified by N-propionyl-L-carnosine, N-butyryl-L-carnosine, N-(2-methylpropionyl)-L-carnosine, N-pentanoyl-L-carnosine, N-hexanoyl-L-carnosine, N-heptanoyl-L-carnosine, and their methyl and ethyl esters. Above mentioned N-alkanoyl-L-carnosine derivatives (IV) may be known compounds or can be synthesized from L-carnosine (I) by per se known methods using acylating or esterifying agents.

The bestatine derivatives represented by the above formula (V) are exemplified by bestatin and its esters such as methyl ester, ethyl ester and propyl ester.

In addition to the above carnosinase inhibitors (II, III, IV and V), the following compounds may also be used for the purpose of the present invention: α-amino acids and their derivatives such as L-alanine, L-leucin, L-isoleucin, L-valine, L-methionine, L-phenylalanine, N-acetyl-L-cysteine, N-aminoacyl-L-histidine (e.g. Gly-L-His, L-Ala-L-His, L-Val-L-His, L-Leu-L-His, L-Ileu-L-His, L-Met-L-His, etc.), N-hydroxy-α-amino acids; γ-amino acids (e.g. γ-aminobutyrylhistamine); thiol type reducing agents (e.g. unithiol, dithiothreitol and 2-mercaptoethanol); manganese ion; zinc ion; substrates of prolinase (L-proline-containing peptides such as L-Pro-His and its derivatives); metal chelating agents (e.g. EDTA); 1,10-phenanthroline; butyl malonate; and so on.

Furthermore, some hydrophobic compounds such as vitamin A and vitamin E with the branched hydrocarbon residue can also be used as the carnosinase inhibitor(s).

As L-carnosine and its related substances, namely one of the active ingredients in this invention, natural substances such as L-carnosine, L-anserine, L-balenine, L-homocarnosine, carcinine and their acyl derivatives (e.g. N-acetyl-L-carnosine, N-acetyl-L-anserine, N-acetyl-L-homocarnosine, etc.) are exemplified. The acyl derivatives may be prepared by per se known method using acylating agents.

As L-carnosine is the major β-alanine source in the human body, its hydrolysis is of metabolic importance. In humans, this reaction is catalyzed by two isozymes: tissue (cytosolic) carnosinase and serum carnosinase. The tissue enzyme is a zinc dependent metalloprotein, and, interestingly, it can be stabilized by other divalent metal cations. Thus the replacement of Zn(II) in the enzyme by some other divalent cations maintains or increases the enzyme activity in vitro. The apparent efficiency of activation by the divalent metal ions is given by the order Cd(II) > Mn(II) >> Zn(II) > Co(II). On the other hand, the enzyme activated by the Mn(II) is inhibited either by cations functioning as activators (e.g. Zn(II), Co(II)) or by non-activating ions such as Be(II) and Fe(II). Cu(II) does not inhibit the enzyme due to the stability of the copper complex of the substrate. Thus the easiest way to protect L-carnosine or its related natural substances from hydrolysis by tissue or serum carnosinase for nutritional purpose would be the combination of the oral L-carnosine with Zn(II) or Mn(II) ion. Oral daily dose of about 400 mg of L-carnosine or another natural derivative such as anserine and balenine admixed with about 5 mg of Zn(II) or Mn(II) is generally recommended for adult human, although the doses can be varied with the purpose of the use. Additionally, vitamin E (40-100 -IU) can be added to the same oral formulation to further increase the bioavailability of L-carnosine.

A unique feature of serum carnosinase should be noted. It is present only in higher primates (man and the great apes) but not in most other mammals (M.C. Jackson et al., Clin. Chim. Acta, Vol. 196, 193, 1991). Accordingly, the purpose of combined application of L-carnosine or its related natural substances with carnosinase inhibitor(s) in equines and canines is to increase the bioavailability of L-carnosine or its related substances in tissues by inhibiting tissue carnosinase in these species.

The carnosinase inhibitors (II), (III), (IV), (V) or others mentioned above may be administered either at the same time or just before or after the administration of L-carnosine or its related substances, or as a composition combined with these compounds.

The carnosinase inhibitors and L-carnosine or its related natural substances or a composition containing thereof may be administered in the form of proper formulations such as powder, granules, capsules, tablets, injection, instillation, lozenges, solutions, ointment, cataplasm, lotion, cream, spray, nasal spray, nasal drops, eye drops, suppository, etc. depending on the purpose of the use. These formulations can be made according to per se known methods by adding properly selected additives and diluents.

In order to prepare liquid formulations suitable for oral use, additives such as water; sugars (e.g. sucrose, sorbit, fructose); glycols (e.g. polyethylene glycol, propylene glycol); oils (e.g. sesame oil, olive oil, soy oil); and preservatives (e.g. p-hydroxybenzoic acid esters) can be used.

In order to prepare solid formulations (e.g. capsules, tablets, powders, granules), diluents (e.g. lactose, glucose, sucrose, mannit); collapsing agents (e.g. starch, sodium alginate); smoothers (e.g. magnesium stearate, talc); binders (e.g. polyvinyl alcohol, hydroxypropyl cellulose, gelatin); surfactants (e.g. fatty acid esters); and plasticizers (e.g. glycerin) can be used.

Among the formulations suitable for parenteral treatment, a solution formulation for intra-venous injection or instillation, may be prepared by using an aqueous medium which is preferably osmometrically equal to the living body. Saline, glucose solution or a mixture thereof may be used as such medium together with suitable additives to prepare a solution, suspension or dispersion by the usual methods.

Eye drops can be prepared by using an aqueous solvent such as a sterile purified water or saline, or by using a non-aqueous solvent such as vegetable oil.

In order to prepare suppository for intestinal application, carriers such as cocoa butter, hydrogenated fat or hydrogenated carboxylic acid can be used.

Spray formulations can be prepared by using carriers which do not irritate the mucous membranes in the mouth and the respiratory tracts, and make the compounds in this invention distribute well as fine particles to accelerate their absorption from the mucous membranes. Such carriers are exemplified by lactose and glycerin. Depending on the properties of the compound in this invention, aerosol or dry powder formulations may be prepared.

In order to prepare formulations for non-oral treatment, one or more than two additives selected from diluents, essences, preservatives, excipients, disintegrants, smoothers, binders, surfactants, plasticizers, etc.

Ointment formulation may be prepared by mixing the compounds in this invention with suitable base such as oily base (e.g. Vaseline, liquid paraffin, silicone, vegetable oil), emulsion base (e.g. water-miscible Vaseline, purified lanolin, and water-soluble base (e.g. macrogol). Emulsifying agents (e.g. negative ionic or non-ionic surfactant) and preservatives (e.g. p-hydroxybenzoic acid esters) may also be added in the ointment.

Other formulations to apply the compounds of this invention to the skin as exemplified by patches, pap, creams, hard ointments, tapes, lotions, solutions, suspensions, emulsions and sprays may be prepared.

In order to enhance the absorption of L-carnosine and the carnosinase inhibitor, it is preferable to add at least one additive selected from cellulose derivative (e.g. hydroxypropylcellulose), gelatin and polyvinylpyrrolidone. The form as pharmaceutical and the method of its preparation in this invention are not limited to the above mentioned explanation.

L-Carnosine (or its related substance) and a carnosinase inhibitor can be added to alcohol-containing drinks such as, whisky, cognac, vodka, beer, sake, and wine to protect the drinkers from various damages of the intestinal tract, the liver, muscles and the brain.

The daily dosage of L-carnosine or its related substances varies with the purpose of its use and the route of its administration but its oral dose is generally in the range of about 0.1 to 200mg per kg body weight, preferably about 0.5 to 100mg per kg body weight, more preferably about 1 to 50mg per kg body weight. The dose, if necessary, may be divided into several times a day. Its dose for injection or instillation is usually about one fifth to one twentieth of the dose of the above oral dose. In the case of formulations for external application, about 0.1 to 20%(w/w), preferably about 0.5 to 10%(w/w), more preferably about 1 to 5 %(w/w) of L-carnosine or its related substance may be included in the formulations. The amount of carnosinase inhibitors to be used simultaneously is in the range of about 0.1 to 20 mole equivalent, preferably about 0.5 to 10 mole equivalent, more preferably about 1 to 5 mole equivalent per mole of L-carnosine. The doses of L-carnosine or its related natural substance and a carnosinase inhibitor to be administered to mammals other than humans are similarly in the dose range for humans as mentioned above.

According to the method of the present invention, L-carnosine or its related substances administered to humans and other mammals can be effectively delivered to and distributed in the plasma and the target tissues or organs without degradation by carnosinase.

### Best Mode for Carrying Out the Invention

The following examples are shown to illustrate the scope of this invention.

### Example 1: Hydrolysis of L-carnosine and related substance with a tissue carnosinase in the absence or the presence of a carnosinase inhibitor.

### Materials and Methods:

Partially purified tissue carnosinase as a solution in 0.1M phosphate buffer (pH7.0) is prepared from the crude brain extracts of male Hairless rats by a reported procedure (Y. Courbebaisse, G. Langrand, J-F. Nicolay and M.A. Babizhayev, Proceeding of 19th IFSCC Congress, Australia, Vol. 3, 1996, p.1-12; M.A. Babizhayev et al., Letters in Peptide Science, Vol.5, 163, 1998). A solution of the substrate (450µl) in 0.1M phosphate buffer (pH7.0) is incubated with the diluted enzyme solution (50µl) at 37°C for 120min. in the absence or in the presence of a carnosinase inhibitor. Portions of the reaction mixture are taken at the time points of 0, 2, 3, 4, 5, 10, 20, 40, 60 min and dansylated with dansyl chloride (5 mg of dansyl chloride/ml of acetonitrile solution) at 50ºC for 15min. The each portion is subjected to HPLC analysis on a column C18DB using a gradient method (A=0.02N acetic acid/acetonitrile, 90/10; B=acetonitrile). The rate of the peptide hydrolysis is determined by following the decrease of the area under the curve (AUC) of the dansylated substrate.

### Results:

The rates of hydrolysis are as follows:
L-Carnosine (0.3mM) : 78 × 10⁻⁶ mole/min (blank test)
L-Carnosine (0.3mM) + β-alanine (0.5 mM): 54 ×10⁻⁶ mole/min
L-Carnosine (0.08 mM): 34 × 10⁻⁶ mole/min (blank test)
L-Carnosine (0.08mM) + L-carnosine (0.3mM) : 78 × 10⁻⁶ mole/min (blank test)
L-Carnosine (0.08mM) + β-alanine (0.5 mM): 5 × 10⁻⁶ mole/min
L-Carnosine (0.08mM) + Gly-L-His (0.5mM): 6 × 10⁻⁶ mole/min
L-Carnosine (0.08mM) + γ-aminobutyrylhistamine (0.5mM): 4.5 × 10⁻⁶ mole/min
L-Carnosine (0.08mM) + L-Pro-L-His (0.5mM): 9 × 10⁻⁶ mole/min
L-Carnosine (0.08mM) + N-acetyl-β-alanine (0.5 mM): 3 × 10⁻⁶ mole/min
N-Acetyl-L-carnosine (0.08 mM): 1 x 10⁻⁶ mole/min (blank test)
N-Acetyl-L-carnosine (0.08 mM) + N-acetyl-β-alanine (0.5 mM) : 0.35 x 10⁻⁶ mole/min
L-Carnosine (0.08mM) + bestatine (0.5 nM): 2 × 10⁻⁶ mole/min Conclusion:

As seen in the above results, it appeared that L-carnosine or the related substance was metabolized (hydrolyzed) less quickly in the presence of a tissue carnosinase inhibitor. This observation indicates that the inhibition of the enzyme with tissue carnosinase activity is at least partially responsible for the delay of the intracellular metabolism and for the accumulation of the natural histidine-containing dipeptides, when L-carnosine or a related substance is administered combined with the claimed inhibitor in vivo.

### Example 2: Combined use of L-carnosine and β-alanine in horses

Five thoroughbred horses were fed 3 times/day with β-alanine (100 mg/kg body weight) and L-carnosine (80 mg/kg body weight) for a period of 25 days. In a separate study, another group of 5 thoroughbred same bred horses were treated similarly with L-carnosine (80mg/kg body weight) only for the same period of 25 days. Percutaneous biopsies of the m. gluteus medius from a depth of 6.2 cm were taken on the days immediately before and after the supplementation period. Heparinized blood samples were also collected at hourly intervals on the first and last days of supplementation, and 2.5 h after ration on every fifth day. Individual muscle samples were dissected from freeze-dried biopsies, weighed and characterized. The concentrations of β-alanine and L-carnosine in plasma and muscles were measured by reported methods (J. J. O'Dowd et al., Biochim. Biophys. Acta, Vol. 967, 241, 1988; M. Dunnett and R.C. Harris, J. Chromatography B, Vol. 688, 47, 1997). The areas under the plasma concentration-time curves (AUC) for β-alanine and L-carnosine were calculated as indicators of the doses absorbed. Muscle fibre carnosine concentrations increased from 60.5- 105.5 mmol/kg (means) ± (S.D. equal to 5-8% of the mean) dry weight in L-carnosine fed horses to 88.9-125.5 mmol/kg (means) ± (S.D. equal to 4-6 % of the mean) dry weight in β-alanine + L-carnosine fed animals. The increases were statistically significant for the average data in 4 of the 5 horses in each group (P<0.05). Changes in muscle L-carnosine concentration appeared to be affected by the bioavailability of β-alanine. Individual increases in muscle L-carnosine concentration were significantly correlated with individual changes in areas under the plasma -concentration time curves (r= 0.895, P < 0.05) of β-alanine.

### Discussion:

It was reported that the increased muscle L-carnosine concentrations correlated with the increased muscular daily activity of horses adapted to either high-speed running or to prolonged periods of hypoxia (M. Dunnett and R.C. Harris, J. Chromatography B, Vol. 688, 47, 1997). Under these conditions, the muscle contraction is known to be reliant upon the anaerobic glycolysis for rapid ATP turnover and to be associated with a large increase in muscle lactic acid concentration. The accumulation of H⁺ from lactic acid dissociation within skeletal muscle would produce a critical fall in intra-cellular pH in the absence of endogenous H⁺ buffering substances. Progressive intracellular acidosis may be a primary cause of local muscle fatigue. The imidazole dipeptides (pKₐ 6.8-7.1) function as important H⁺ buffers over the physiological pH range (R.C. Harris et al., Comp. Biochem. Physiol., Vol.97A, 249, 1990). Higher L-carnosine concentrations in muscle fibres are consistent with their higher capacity for H⁺ production and thus greater buffering requirement (M. Dunnett and R.C. Harris, J. Chromatography B, Vol. 688: 47, 1997). The combined oral administration of L-carnosine with β-alanine, an inhibitor of carnosinase, is a suitable way to increase the concentration of the imidazole-containing dipeptide, L-carnosine, in the muscular tissues.

### Example 3: Induction of apoptosis in the cancer cells upon application of L-carnosine with a carnosinase inhibitor

The experimental design, details of experimental procedure and human leukemia cell lines were essentially the same as those reported by Sekine et al. (Int. J. Cancer, Vol. 94, 485, 2001). The carnosinase inhibitor bestatine was used in the range of 5-15 µg/ml of concentration and the concentration of the applied carnosine varied from 5-20 mM . The combined use of L-carnosine (10 mM) and the tissue carnosinase inhibitor bestatin (10 µg/ml) induced apoptosis in several human leukemia cell lines. Our study was performed with this combination to examine whether bestatin + carnosine can also induce apoptosis in solid tumor cell lines. Bestatin alone at above-cited concentrations exhibited neither direct growth inhibition nor induction of apoptosis in the tumor cell lines examined. However, the composition of L-carnosine + bestatin significantly augmented the growth-inhibitory effect and induction of apoptosis to the cited tumor cell lines. When used with L-carnosine, bestatin methyl ester, a more cell-permeable bestatin derivative with similar inhibitory activity against cytosolic tissue carnosinase, inhibited cell growth more clearly than the case where only L-carnosine is used. In our studies according to the above-cited design, the combination of 10 µg/ml of bestatin with 10 mM L-carnosine promoted processing of caspase 3 to the active form p17 and efflux of mitochondrial cytochrome C into the cytosol in solid tumor cells and in several human leukemia cell lines.

### Conclusion:

These results suggest that intracellular administration of carnosine + carnosinase inhibitor might play an important role in the apoptosis of the solid tumor and other cell lines

### Example 4: Addition of L-carnosine and a carnosinase inhibitor to the alcoholic drink

We have used the newly composed Extra Vodka Corn including L-carnosine and β-alanine which was manufactured at the Russian Plant Corn & Co. by making a solution of 70 mg/200 g of each ingredient in the vodka with vigorous stirring. The taste of and the euphoria properties of the vodka unchanged, and no side reactions like headache or liver toxicity were observed upon 20 days of the assumption by the Managers of this Plant who taste the vodka drink regularly. Five volunteers used the composed vodka drink each day at a dose of 200 g. The level of acetaldehyde and malonaldehyde were not increased in the urine at the end of the trial on the 20^{th} day in the morning one hour after drink as compared to those measured prior to the study.

### Example 5: Oral care application of L-carnosine with a carnosinase inhibitor

L-Carnosine and β-alanine were applied to 7 men subjects with gingivitis as a combined, oral formulation. The composition of carnosine + β-alanine was found to stimulate the healing of gingival tissues and completely abolished bleeding. The reaction of inflammation was decreased. The cited composition of carnosine 5 mg/ml + β-alanine (7 mg/ml) demonstrated significantly increased granulation in the tooth gingival tissues. The data on the use of L-carnosine only for the wound healing of gingival tissues have been published earlier (K. Nagai, Langenbecks Arch. Chir., Vol. 351, 39, 1980; T. Yamane et al., J. Nihon Univ. School. Dent., Vol. 19, 70, 1977).

### Example 6. Application of L-carnosine with the carnosinase inhibitor β-alanine in cosmetic composition as a pigment tanning product

This composition can induce the pigment adaptation in dark brown, blond and pigmented hair and in the skin.
The cosmetic composition of β-alanine with carnosine was heated with phospholipid lecithin to produce the sun tanning pigment in the skin. β-Alanine does not produce the coloring in the solution per se and can produce the coloring by the reactions with tyrosine and tyrosinase-oxidized tyrosine, dopa or dopamine.

Preparation of the cosmetic formulation (described in : Babizhayev M.A. Antioxidant activity of L-carnosine, a natural histidine-containing dipeptide in crystalline lens. Biochim. Biophys. Acta 1004 (1989) 363-371):

A mixture of 1% carnosine and 2 % β-alanine residues were included in the liposomes of lecithin (derived from egg yolk phosphatidylcholine) prepared by reverse phase evaporation. The liposomes were formed from an emulsion of phospholipid in the phosphate buffered solution loaded with the cited compounds with a modified procedure including the two steps simultaneously, i.e., mixing of heptane and buffer solution and removal of organic solvent. In a standard preparation 100 mg of phosphatidylcholine was dissolved in 15 ml heptane in a round-bottomed flask and 5 ml of 0.1 mol/l (or less concentrated) PBS buffer (pH 7.4) was added. The flask was immersed in a water-bath warmed to 50ºC. After thorough mixing nitrogen was poured through a glass capillary into the sample. Heptane was usually removed at a rate of 0.5-1.0 ml· min⁻¹. We observed an enhancement of the viscosity of the mixture up to a paste-like state. After that, the gel collapsed relatively fast and a turbid nonviscous liposome suspension was formed. Single gel droplets disappeared after further nitrogen pouring. The mean hydrodynamic diameter of vesicles in the prepared cosmetic composition was 350 nm with a polydispersity of 27%.

The composition was daily applied on the skin of human elderly volunteers (10 subjects) for 15 days twice daily and showed pigmentary adaptation to produce a smooth tan pigment including the disappearance of age-related lipofuscin-like spots on the skin. The spots were present originally in the volunteers or were then were substituted with the tanning pigment upon the treatment. The fluorescence of the skin in the blue green zone (measured with spectrofluorophotometer equipped with a distant opto-fiber probe) was decreased and good/fresh appearance of the skin increased. Both spectra of excitation and emission of fluorescence at 365 nm excitation wavelength were monitored and the intensity of fluorescence of lipofuscin-associated pigments was decreased. The subjects underwent with the cited treatment reported the decrease of the spot lipofuscin-like age pigments previously exposed on the skin. The pure carnosine application showed only a little whitening activity during the experiment. The observed disappearance of lipofuscin-related spots on the skin is related to the action of carnosine as the de-linker of cross-links in the skin proteins combined with the tanning activity of β-alanine.

### Example 7: Treatment of autism

For autistic children, most beneficial effects were obtained when treated orally with a dosage of 400 mg L-anserine in combination with 50-IU Vitamin E and 5 mg zinc twice a day. In some children, too high dose may overstimulate some patient's frontal lobes which may cause increased irritability, hyperactivity or insomnia which was observed already in hyperactive autistic children.

### Example 8: Treatment of neural and brain disorders

Children with neural disorders such as epilepsy, central processing disorder, or brain injury were able to be treated with oral daily dose of 200 to 2000mg of L-carnosine in combination with 40- IU to 150- IU Vitamin E and 4 to 15 mg zinc.

### Example 9

The following formulations are made by per se ordinary procedures:

### 1. Cosmetic formulation:

A hydroglycolic solution of which analytical composition is :

| | |
|---|---|
| Carnosine | 10.000 g |
| β-alanine | 15.000 g |
| 1,3-Butanediol | 8.180 g |
| Sodium methyl paraben | 0.145 g |
| Water up to | 100.000 g |

The composition is a limpid liquid, colorless, with a light butanediol odor which is miscible with water, glycols and alcohol, but not miscible with hexane, mineral and vegetable oils.

### 2. Oral formulation for dietary supplement:

Capsulated formulation in one gelatine or rice capsule:

| | |
|---|---|
| L-Carnosine | 80 mg |
| β-alanine | 100 mg |

### 3. Ophthalmic formulation for oxidative stress-induced ocular disorders:

| | |
|---|---|
| L-Carnosine | 1.00 g |
| β-alanine | 1.00 g |
| Dibasic sodium phosphate | 0.80 g |
| Monobasic sodium phosphate | 0.15 g |
| Benzalconium chloride | 0.010 g |
| Purified water up to | 100 ml |

### 4. Alcoholic drink:

| | |
|---|---|
| 40% Vodka | 500 ml |
| L-Carnosine | 400 mg |
| β-alanine | 600 mg |
| Water | up to 1000 ml |

### Industrial Applicability

The present invention aims to provide combined use of a carnosinase inhibitor with L-carnosine and its related substance and a composition containing the same, which are useful for treatment or prevention of diseases and improvement of health conditions. The present invention also provides a method for prevention or treatment of carnosine-related diseases, a method for improvement of health conditions, a method for improvement of exercise performance, a method for improvement of skin health and a method for prevention of the side effects of alcohol drinking, comprising combined use of a carnosinase inhibitor with L-carnosine and its related substance, for mammals. The present invention is useful in the fields of pharmaceuticals, cosmetics, health foods, foods and beverages.

## Claims

1. Combined use of a carnosinase inhibitor with L-carnosine and its related substance to treat or prevent carnosine-related disorders and diseases of humans and other mammals.

2. Combined use of a carnosinase inhibitor with L-carnosine and its related substance to improve health conditions of humans and other mammals.

3. Combination of a carnosinase inhibitor with L-carnosine or its related substance to improve health conditions of the skin of humans and other mammals.

4. Combined use of a carnosinase inhibitor with L-carnosine or its related substance to prevent adverse effects caused by drinking alcohol.

5. The combined use according to Claim 1 to 4, wherein at least one of the carnosinase inhibitors selected from the group comprising of β-alanine (II), an N-alkanoyl-β-alanine derivative (III), an N-alkanoyl-L-carnosine derivative (IV) and bestatin (V) is used. 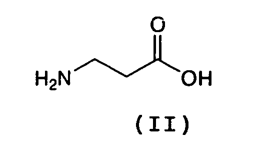 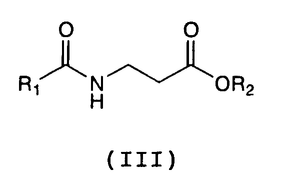 (R₁ and R₂ independently represent hydrogen atom or a lower alkyl group) 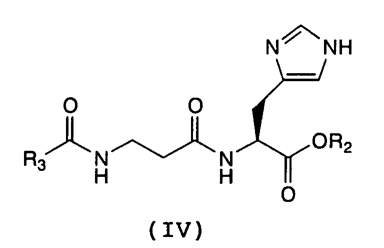 (R₂ represents hydrogen atom or a lower alkyl group, and R₃ represents an alkyl group
of more than two carbon atoms) 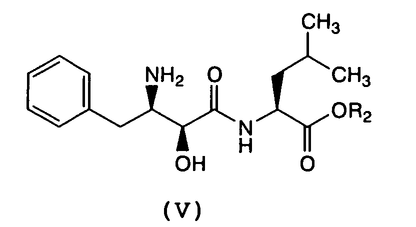 (R₂ represents hydrogen atom or a lower alkyl group).

6. The combined use according to Claims 1 to 4 to increase the concentration of L-carnosine or its related substance in the plasma and the cells of humans and other mammals to the physiologically effective levels.

7. Combined use according to Claims 1 to 4, wherein the L-carnosine-related substance is N-acetyl-L-carnosine, L-anserine, N-acetyl-L-anserine, L-balenine, L-homocarnosine, N-acetyl-L-homocarnosine or carcinine.

8. The combined use according to Claims 1 to 4, the carnosinase inhibitor is β-alanine.

9. The combined use according to Claim 5, wherein N-alkanoyl-β-alanine (III) is N-acetyl-β-alanine which may be esterified by a lower alkyl group.

10. The combined use according to Claims 1 to 4, wherein at least one of the carnosinase inhibitors selected from the group comprising of an α-amino acid derivative, γ-amino acid derivative, a thiol type reducing agent, a metal chelating agent, a hydrophobic vitamin with a branched hydrocarbon residue, manganese ion or zinc ion is used.

11. The method of increasing the concentration of L-carnosine or its related substance in the plasma and the cells to the physiologically effective level, wherein at least one of the carnosinase inhibitors selected from the group comprising of β-alanine (II), an N-alkanoyl-β-alanine derivative (III), an N-alkanoyl-L-carnosine derivative (IV), bestatin (V), an α-amino acid derivative, a γ-amino acid derivative, a thiol type reducing agent, a metal chelating agent, a hydrophobic vitamin with a branched hydrocarbon residue, manganese ion and zinc ion.

12. The method according to Claim 11, wherein N-acetyl-L-carnosine, L-anserine, N-acetyl-L-anserine, L-balenine, L-homocarnosine, N-acetyl-L-homocarnosine, or carcinine is used as an L-carnosine-related substance.

13. The method according to Claim 11, the carnosinase inhibitor is β-alanine.

14. A pharmaceutical composition containing L-carnosine or its related natural substance and at least one of the carnosinase inhibitors selected from the group comprising of β-alanine (II), an N-alkanoyl-β-alanine derivative (III), N-alkanoyl-L-carnosine derivative (IV), bestatin (V), an α-amino acid derivative, a γ-amino acid derivative, a thiol type reducing agent, a metal chelating agent, a hydrophobic vitamin with a branched hydrocarbon residue, manganese ion and zinc ion.

15. A health food or a dietary supplement composition containing L-carnosine or its related natural substance and at least one of the carnosinase inhibitors selected from the group comprising of β-alanine (II), an N-alkanoyl-β-alanine derivative (III), an N-alkanoyl-L-carnosine derivative(IV), bestatin (V), an α-amino acid derivative, a γ-amino acid derivative, a thiol type reducing agent, a metal chelating agent, a hydrophobic vitamin with branched hydrocarbon structure, manganese ion and zinc ion.

16. A health food or a dietary supplement composition according to Claim 15, wherein the carnosinase inhibitor is β-alanine.

17. A cosmetic or a skin-care composition containing L-carnosine or its related natural substance and at least one of the carnosinase inhibitors selected from the group comprising of β-alanine (II), an N-alkanoyl-β-alanine derivative (III), N-alkanoyl-L-carnosine derivative (IV), bestatin (V), an α-amino acid derivative, γ-amino acid derivative, a thiol type reducing agent, a metal chelating agent, a hydrophobic vitamin with branched hydrocarbon structure, manganese ion and zinc ion.

18. Alcoholic drinks containing L-carnosine or its related natural substance and a carnosinase inhibitor selected from the group comprising of β-alanine (II), an N-alkanoyl-β-alanine derivative (III), N-alkanoyl-L-carnosine derivative (IV), bestatin (V), an α-amino acid derivative, γ-amino acid derivative, a thiol type reducing agent, a metal chelating agent, a hydrophobic vitamin with branched hydrocarbon structure, manganese ion and zinc ion.

19. The composition according to Claims 14 to 17, wherein at least one of the additives selected from the group comprising of a cellulose derivative, gelatine and polyvinyl pyrrolidone is used to increase the absorption of L-carnosine or its related substance and/or the carnosinase inhibitor.

20. The composition according to Claims 14 to 17, wherein N-acetyl-L-carnosine, L-anserine, N-acetyl-L-anserine, L-balenine, L-homocarnosine, N-acetyl-L-homocarnosine, or carcinine is used as an L-carnosine-related substance.

21. The composition according to Claims 14 to 18, wherein the carnosinase inhibitor is β-alanine.

22. Cancer chemotherapy **characterized by** treating cancer patients with L-carnosine or its related substance together with bestatin (V).
